(19) Europäisches Patentamt
European Patent Office
Office européen des brevets

(11) Veröffentlichungsnummer: **0 089 557**
**B1**

# (12) EUROPÄISCHE PATENTSCHRIFT

(45) Veröffentlichungstag der Patentschrift: **10.07.85**

(51) Int. Cl.⁴: **C 07 C 102/06**, C 07 C 103/64

(21) Anmeldenummer: **83102335.3**

(22) Anmeldetag: **10.03.83**

(54) **Verfahren zur Herstellung von N-(tert.Aminoalkyl)acrylamiden.**

(30) Priorität: **18.03.82 DE 3209800**

(43) Veröffentlichungstag der Anmeldung: **28.09.83 Patentblatt 83/39**

(45) Bekanntmachung des Hinweises auf die Patenterteilung: **10.07.85 Patentblatt 85/28**

(84) Benannte Vertragsstaaten: **AT BE CH DE FR GB IT LI LU NL SE**

(56) Entgegenhaltungen:
**EP-A-0 013 416**
**DE-A-2 816 516**

**Die Akte enthält technische Angaben, die nach dem Eingang der Anmeldung eingereicht wurden und die nicht in dieser Patentschrift enthalten sind.**

(73) Patentinhaber: **Chemische Fabrik Stockhausen GmbH, Bäkerpfad 25, D-4150 Krefeld (DE)**

(72) Erfinder: **Mertens, Richard, Dr., Dipl.-Chem., Bönnersweg 12, D-4150 Krefeld (DE)**
Erfinder: **Dahmen, Kurt, Dr., Dipl.-Chem., von-Velsen-Strasse 6, D-4050 Mönchengladbach (DE)**

(74) Vertreter: **Klöpsch, Gerald, Dr.-Ing., An Gross St. Martin 6, D-5000 Köln 1 (DE)**

**EP 0 089 557 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung bezieht sich auf ein einfaches Verfahren zur Herstellung von N-(tert.-Aminoalkyl)-acrylamiden, die in Homo- und Copolymerisationen wichtige Einsatzmöglichkeiten finden, die zu Homo- bzw. Copolymerisaten führen, welche als Flockungs- und Sedimentationshilfsmittel sowie als Retentionshilfsmittel in der Papierindustrie gut geeignet sind.

Zur Herstellung von N-(tert.-Aminoalkyl)acrylamiden sind in der Literatur eine Reihe von Verfahren bekannt. So ist in der DE-OS 2 502 247 angegeben, daß bei der Umsetzung von Acrylsäure bzw. Acrylsäureestern mit einem doppelmolaren Überschuß an N,N-Dialkylalkylendiaminen die entsprechenden $\beta$-Aminopropionsäureamide entstehen, aus denen durch thermische $\beta$-Eliminierung die N-(tert.-Aminoalkyl)acrylamide hergestellt werden können. Die notwendig hohen Pyrolysetemperaturen dieses Verfahrens fördern jedoch nachteilig die Bildung von Nebenprodukten sowie die Polymerisation der Acrylamide. Weiterhin erfordert die Reinigung der Crack-Produkte eine aufwendige Destillationstechnik. So gestaltet sich diese zweistufige Arbeitsweise sehr unbefriedigend.

Bei den Verfahren der DE-OS 2 809 102 und 2 816 516 werden die Ester der (Meth)acrylsäure mit einem Unterschuß oder nur geringen Überschuß an N,N-Dialkylalkylendiamin in Gegenwart von Dialkylzinnoxid bzw. Eisenionen zu den N-substituierten (Meth)acrylamiden umgesetzt. Bei diesem Verfahren werden die erwünschten (Meth)acrylamide jedoch nur in völlig unzureichenden Ausbeuten erhalten. Zudem muß nach der DE-OS 2 809 102 unter Druck gearbeitet werden.

Die US-Patentschrift 3 652 671 beschreibt ein Verfahren zur Herstellung von N-(Dialkylaminoalkyl)methacrylamiden, bei dem Methacrylsäure und N,N-Dialkylalkylendiamine in äquimolaren Mengen einer erhöhten Temperatur unterworfen werden. Aus dem Salz bildet sich sehr schnell das Michael-Addukt, d. h. das N-(Dialkylaminoalkyl)-2-methyl-$\beta$-alanin, das sich bei 140°—230° umlagert zum N-(Dialkylaminoalkyl)-methacrylamid. Als sehr nachteilig muß angesehen werden, daß nur N-substituierte Methacrylamide aus den entsprechenden Methacrylsäure-Addukten hergestellt werden können und daß Acrylsäureamide nach diesem Verfahren nicht herstellbar sind. Es wird in der Patentschrift angegeben, daß beim Einsatz von Acrylsäure Nebenreaktionen vorherrschen und zum größten Teil teerartige Materialien entstehen. Von den gewünschten N-substituierten Acrylamiden können nur geringe Mengen isoliert werden.

Es wurde nun überraschenderweise gefunden, daß N-(Dialkylaminoalkyl)acrylamide der allgemeinen Formel

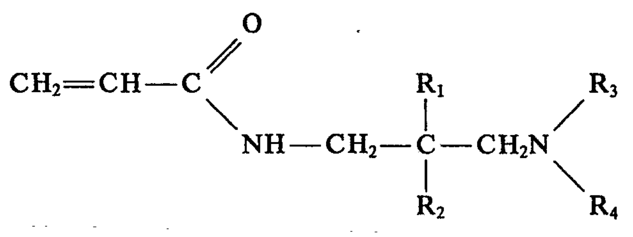

(I)

worin $R_1$, $R_2$, $R_3$ und $R_4$ jeweils gerad- oder verzweigtkettige Alkylgruppen mit 1—4 C-Atomen sind, in guten Ausbeuten hergestellt werden können, wenn man Acrylsäure mit einem tertiären Aminoalkylamin der Formel

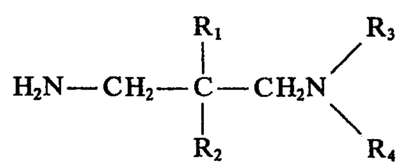

(II)

worin $R_1$ bis $R_4$ die oben angegebene Bedeutung haben, in äquimolaren Mengen von Acrylsäure und Aminoalkylamin bei Temperaturen von 120—300°C, vorzugsweise 150—230°C, ggf. in Gegenwart katalytischer Mengen saurer oder basischer Substanzen umsetzt. Vorzugsweise wird das bei der Reaktion entstehende Wasser abdestilliert.

Für die Erfindung besonders geeignete tertiäre Aminoalkylamine der Formel II: 3-Dimethylamino-2,2-dimethylpropylamin, 3-Diäthylamino-2,2-dimethylpropylamin, 3-Dimethylamino-2,2-diäthylpropylamin und 3-Diäthylamino-2,2-diäthylpropylamin, von denen 3-Dimethylamino-2,2-dimethylpropylamin besonders bevorzugt wird.

Die Durchführung der Reaktion gestaltet sich sehr einfach. Zu dem N,N-Dialkylalkylendiamin, das gegebenenfalls einen Polymerisationsinhibitor und gegebenenfalls einen Katalysator enthält, wird die Acrylsäure zugetropft, wobei die Temperatur auf über 100°C ansteigen kann. Nach beendeter Zugabe wird die Mischung auf 150°—230° erhitzt, wobei innerhalb von 1—5 h die theoretische Menge Wasser abdestilliert. Das gebildete Acrylamid wird durch Vakuumdestillation isoliert. Die N-(tert.-Aminoalkyl)acrylamide entstehen in guten Ausbeuten und können ohne weitere Reinigung in Homo- oder Copolymerisationen eingesetzt werden.

Obwohl für den Ablauf und die Ausbeuten nicht unbedingt notwendig, hat es sich als vorteilhaft erwiesen, katalytische Mengen alkalischer oder saurer Substanzen in einer Menge von etwa 0,5 bis 2 Gew.-% — bezogen auf das Gesamtgewicht der Reaktanten — zuzusetzen. Diese können sowohl anorganischer Natur, wie z. B. Salzsäure, Phosphorsäure, Kaliumcarbonat und Kaliumhydroxid als auch organischer Natur sein wie Essigsäure, Natriumacetat oder Alkoholate. Ebenso können Lewis-Säuren und Basen oder Ionenaustauscher verwendet werden. Bevorzugt werden Phosphorsäure und Bortrifluorid-Ätherat eingesetzt.

Die Reaktion kann in Gegenwart der üblichen Polymerisationsinhibitoren durchgeführt werden, wie z. B. Hydrochinon, p-Methoxyphenol, Kupferpulver oder Kupfersalze. Bevorzugt werden aromatische Amine, wie z. B. N-Phenyl-$\beta$-naphthylamin, N,N'-Diphenyl-p-phenylendiamin oder Phenothiazin.

## Beispiel 1

Ein 500-ml-Dreihalskolben, ausgestattet mit Magnetrührer, Thermometer und einer kurzen Kolonne mit Destillationsaufsatz, wird mit 260 g (2 Mol) 3-Dimethylamino-2,2-dimethylpropylamin, 4 g N-Phenyl-$\beta$-naphthylamin und 4 ml 85%iger Phosphorsäure beschickt. 144 g (2 Mol) Acrylsäure werden in 0,5 h zugetropft, wobei die Temperatur der Mischung bis auf 150°C ansteigt. Anschließend wird die Reaktionsmischung unter Stickstoff innerhalb von 2 h auf 200°C erhitzt und 1 h bei dieser Temperatur gerührt. Währenddessen werden 35,5 g Destillat aufgefangen. Nach dem Abkühlen wird im Vakuum destilliert, und es werden 247 g (67%) N-(3-Dimethylamino-2,2-dimethylpropyl)acrylamid, Kp. 110—115° bei 66,5 Pa erhalten.

## Beispiel 2

Wie in Beispiel 1 verfahren, werden 260 g 3-Dimethylamino-2,2-dimethylpropylamin, 144 g Acrylsäure, 4 g N,N'-Diphenyl-p-phenylendiamin und 10 ml $BF_3$-Ätherat vermischt. Es können 265 g (72%) des N-(3-Dimethylamino-2,2-dimethylpropyl)acrylamid isoliert werden.

## Beispiel 3

Wie in Beispiel 1 verfahren, werden 60 g 3-Diethylamino-2,2-dimethylpropylamin, 27,3 g Acrylsäure, 1,0 g N,N'-Diphenyl-p-phenylendiamin und 1 ml 85%iger Phosphorsäure vermischt. Es können 47,5 g (59%) N-(3-Diethylamino-2,2-dimethylpropyl)acrylamid, Kp. 139—141° bei 1,33 Pa isoliert werden.

## Beispiel 4

Wie in Beispiel 1 verfahren, werden 173,5 g 3-Dimethylamino-2-ethyl-2-butylpropylamin, 67 g Acrylsäure, 2 g N,N'-Diphenyl-p-phenylendiamin und 2 g Kalium-tert.-Butylat vermischt. Es können 122 g (55%) N-(3-Dimethylamino-2-ethyl-2-butylpropyl)-acrylamid, Kp. 129° bei 66,5 Pa isoliert werden.

## Beispiel 5

Wie in Beispiel 1 verfahren, werden 130 g 3-Dimethylamino-2,2-dimethylpropylamin, 72 g Acrylsäure, 3 g Kupferazetat und 2 g 85%ige Phosphorsäure vermischt. Es können 130,5 g (71%) N-(3-Dimethylamino-2,2-dimethylpropyl)acrylamid isoliert werden.

**Patentansprüche**

1. Verfahren zur Herstellung von N-(tert.-Aminoalkyl)acrylamiden der allgemeinen Formel

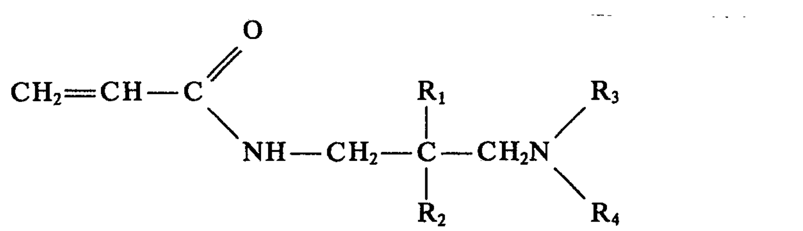

(I)

worin $R_1$, $R_2$, $R_3$ und $R_4$ jeweils eine gerad- oder verzweigtkettige $C_1$- bis $C_4$-Alkylgruppe bedeuten,

dadurch gekennzeichnet, daß Acrylsäure mit einer äquimolaren Menge eines tertiären Aminoalkylamins der allgemeinen Formel

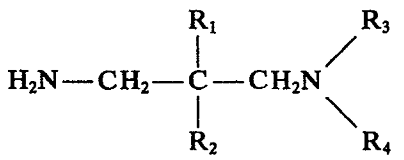

(II)

worin $R_1$, $R_2$, $R_3$ und $R_4$ die gleiche Bedeutung wie in Formel 1 haben, bei Temperaturen von 120—300, vorzugsweise 150—230° C, umgesetzt und das Reaktionsprodukt aus dem Reaktionsgemisch, vorzugsweise durch Destillation, abgetrennt wird.

2. Verfahren nach Anspruch 1, dadurch gekennzeichnet, daß die Umsetzung in Gegenwart katalytischer Mengen saurer oder basischer Substanzen durchgeführt wird.

3. Verfahren nach Anspruch 2, dadurch gekennzeichnet, daß als saure oder basische Substanzen organische oder anorganische Säuren oder Basen, Lewis-Säuren oder -basen oder Ionenaustauscher verwendet werden.

4. Verfahren nach Ansprüchen 1 bis 3, dadurch gekennzeichnet, daß die Umsetzung in Gegenwart von Hydrochinon, p-Methoxyphenol, N-Phenyl-$\beta$-naphthylamin, Phenothiazin, N,N-Diphenyl-p-phenylendiamin, Kupferpulver und/oder Kupfersalzen aus Polymerisationsinhibitoren durchgeführt wird.

**Claims**

1. Process for the manufacture of N-(tert.-aminoalkyl)-acrylamides of the general formula

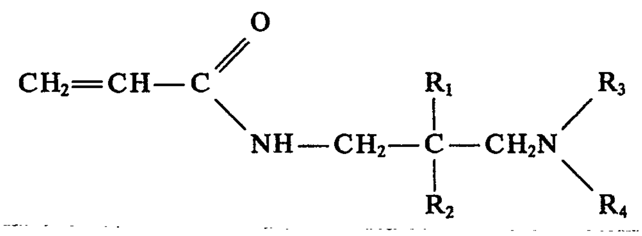

(I)

in which each of $R_1$, $R_2$, $R_3$ and $R_4$ represents a $C_1$- to $C_4$-alkyl group that is a straight or branched chain, characterised in that acrylic acid is reacted with an equimolar amount of a tertiary aminoalkylamine of the general formula

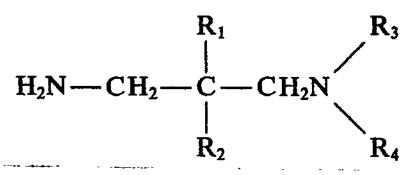

(II)

in which $R_1$, $R_2$, $R_3$ and $R_4$ have the same meanings as in formula I, at temperatures from 120 to 300° C, preferably from 150 to 230° C, and the reaction product is separated off from the reaction mixture, preferably by distillation.

2. Process according to claim 1, characterised in that the reaction is carried out in the presence of catalytic amounts of acidic or basic substances.

3. Process according to claim 2, characterised in that as acidic or basic substances there are used organic or inorganic acids or bases, Lewis acids or bases or ion exchangers.

4. Process according to claims 1 to 3, characterised in that the reaction is carried out in the presence of hydroquinone, p-methoxyphenol, N-phenyl-$\beta$-naphthylamine, phenothiazine, N,N-diphenyl-p-phenylenediamine, copper powder and/or copper salts, in the form of polymerisation inhibitors.

**Revendications**

1. Procédé de préparation de N-(tert.-amino-alcoyl)-acrylamides de la formule générale

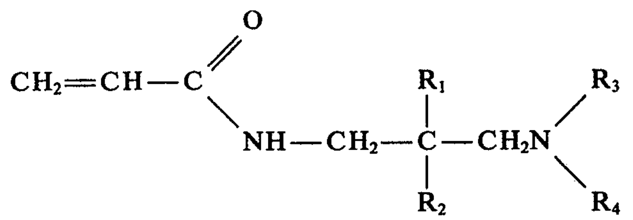

(I)

dans laquelle $R_1$, $R_2$, $R_3$ et $R_4$ désignent chacun un radical alcoyle en $C_1$ à $C_4$ à chaîne droite ou ramifiée, caractérisé en ce que l'on fait réagir l'acide acrylique à des températures comprises entre 120 et 300 et de préférence entre 150 et 230° C avec une proportion équimolaire d'une amino-alcoyl-amine tertiaire de la formule générale

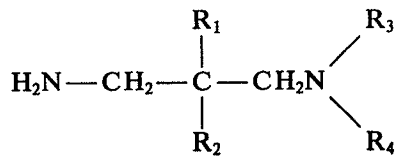

(II)

dans laquelle $R_1$, $R_2$, $R_3$ et $R_4$ possèdent la signification définie pour la formule I, le produit réactionnel étant ensuite séparé du mélange réactionnel, de préférence par distillation.

2. Procédé suivant la revendication 1, caractérisé en ce que la réaction est effectuée en présence de quantités catalytiques de substances acides ou basiques.

3. Procédé suivant la revendication 2, caractérisé en ce que les substances acides ou basiques sont choisies parmi les acides et bases organiques ou inorganiques, les acides ou bases de Lewis et les échangeurs d'ions.

4. Procédé suivant les revendications 1 à 3, caractérisé en ce que la réaction est réalisée en présence d'inhibiteurs de la polymérisation choisis parmi l'hydroquinone, le p-méthoxy-phénol, la N-phényl-$\beta$-naphtylamine, la phénotiazine, la N,N-diphényl-p-phénylènediamine, le cuivre pulvérulent et (ou) les sels du cuivre.